# EUROPEAN PATENT APPLICATION

(11) **EP 2 842 560 A1**
(43) Date of publication of application: **04.03.2015**
(21) Application number: 13782640.0
(22) Date of filing: 23.04.2013
(51) Int. Cl.: A61K 31/702, A61P 1/00, A61P 43/00

(54) **COMPOSITION FOR PROMOTING BIFIDOBACTERIA GROWTH**

(30) Priority: 23.04.2012 JP 2012098224
(71) Applicant: Kyoto University, Sakyo-ku Kyoto-shi Kyoto 606-8501 (JP); Kyowa Hakko Bio Co., Ltd., Tokyo 100-8185 (JP)
(72) Inventor: YAMAMOTO, Kenji, Kyoto-shi Kyoto 606-8501 (JP); KOIZUMI, Satoshi, Tsukuba-shi Ibaraki 305-0841 (JP)
(74) Representative: Grünecker, Kinkeldey, Stockmair & Schwanhäusser
(86) International application number: PCT/JP2013/061930
(87) International publication number: WO 2013/161820

(57) **Abstract**

The present invention provides a growth promoting composition capable of promoting growth of a microorganism belonging to the genus Bifidobacterium living in the intestine of mammals such as human and the like.

According to the present invention, a composition for promoting growth of a microorganism belonging to the genus Bifidobacterium, comprising one or more kinds selected from the group consisting of Lacto-N-tetraose, Lacto-N-neotetraose and derivatives thereof can be provided. The aforementioned growth promoting composition is suitable for promoting growth of the aforementioned microorganism in the intestine by oral administration to, particularly, a human adult.

## Description

### Technical Field

The present invention relates to a composition for promoting growth of intestinal bifidobacteria, comprising oligosaccharide as an active ingredient.

### Background Art

It is known that bifidobacteria is an advantageous enteric bacterium that affords an intestine-regulating function, an antiallergic action, an immunostimulatory action and the like to the host. Since infants fed on breast milk have an overwhelmingly high flora ratio of bifidobacteria in the intestine as compared to those fed on artificial milk, the growth factor thereof has been searched for over many years.

It is known that Bifidobacterium bifidum has an ability to hydrolyze Lacto-N-tetraose (hereinafter to be referred to as LNT) and produce Lacto-N-Biosidase (hereinafter to be referred to as LNB) having an activity to produce Lacto-N-biose, and LNB has a bifidobacteria growth-promoting effect (non-patent documents 1 and 2). It is also known that Lacto-N-neotetraose (hereinafter to be referred to as LNnT) has a Bifidobacterium infantis metabolism-promoting action, which is found in the intestine of infants (patent document 1). However, it is not known that LNT and LNnT promote growth of bifidobacteria, particularly Bifidobacterium bifidum and Bifidobacterium longum living in the intestine of human adult.

### [Document List]

### [patent document]

patent document 1: WO 98/43495

### [non-patent documents]

non-patent document 1: Appl. Environ. Microbiol., 74, 3996-4004, 2008
non-patent document 2: Biosci. Biotechnol. Biochem., 73, 1175-1179, 2009

### SUMMARY OF THE INVENTION

### Problems to be Solved by the Invention

It is therefore an object of the present invention to provide a growth promoting composition that shows good growth-promoting effect on bifidobacteria, particularly B. bifidum and B. longum living in the intestine of human adult, which have advantageous actions such as immunostimulatory action and the like and are known as representative good bacteria from among the enteric bacteria.

### Means of Solving the Problems

The present inventors have conducted intensive studies in an attempt to solve the aforementioned problems and found that oligosaccharides such as Lacto-N-tetraose (LNT) and Lacto-N-neotetraose (LNnT) show a good growth-promoting effect on bifidobacteria, particularly B. bifidum and B. longum living in the intestine of human adult, which resulted in the completion of the present invention.

Accordingly, the present invention relates to the following (1) - (13).
(1) A composition for promoting growth of a microorganism belonging to the genus Bifidobacterium, comprising one or more kinds selected from the group consisting of Lacto-N-tetraose, Lacto-N-neotetraose and derivatives thereof.
(2) The composition of the above-mentioned (1), wherein the microorganism belonging to the genus Bifidobacterium is one or more kinds selected from the group consisting of a microorganism belonging to Bifidobacterium bifidum and a microorganism belonging to Bifidobacterium longum.
(3) The composition of the above-mentioned (1) or (2), which promotes the growth of a microorganism belonging to the genus Bifidobacterium in the intestine of human adult.
(4) The composition of any of the above-mentioned (1) - (3), which is a growth promoter of a microorganism belonging to the genus Bifidobacterium.
(5) The composition of any of the above-mentioned (1) - (3), which is a food or drink.
(6) The composition of the above-mentioned (5), wherein the food or drink is a food with health claims or dietary supplement.
(7) The composition of any of the above-mentioned (1) - (3), which is a food additive.
(8) Use of one or more kinds selected from the group consisting of Lacto-N-tetraose, Lacto-N-neotetraose and derivatives thereof for the production of a composition for promoting growth of a microorganism belonging to the genus Bifidobacterium.
(9) A method of promoting growth of a microorganism belonging to the genus Bifidobacterium in a subject in need thereof, comprising orally administering a composition containing one or more kinds selected from the group consisting of Lacto-N-tetraose, Lacto-N-neotetraose and derivatives thereof to the subject.
(10) The method of the above-mentioned (9), wherein the microorganism belonging to the genus Bifidobacterium is one or more kinds selected from the group consisting of a microorganism belonging to Bifidobacterium bifidum and a microorganism belonging to Bifidobacterium longum.
(11) The method of the above-mentioned (9) or (10), which promotes growth of a microorganism belonging to the genus Bifidobacterium in the intestine of human adult.
(12) A composition comprising one or more kinds selected from the group consisting of Lacto-N-tetraose, Lacto-N-neotetraose and derivatives thereof for use in promoting growth of a microorganism belonging to the genus Bifidobacterium.
(13) A commercial package comprising a composition for promoting growth of a microorganism belonging to the genus Bifidobacterium, which contains one or more kinds selected from the group consisting of Lacto-N-tetraose, Lacto-N-neotetraose and derivatives thereof, and a written matter with an explanation relating to use for promoting growth of a microorganism belonging to the genus Bifidobacterium.

### Effect of the Invention

Oral ingestion of the growth promoting composition of the present invention can efficiently grow a microorganism belonging to the genus Bifidobacterium, particularly a microorganism belonging to Bifidobacterium bifidum or a microorganism belonging to Bifidobacterium longum, in a mammal, particularly in the intestine of human adult. As a result, enteric bacterial flora can be improved to a good state, and the state can be maintained, and bowel diseases and various symptoms caused by changes in the enteric bacterial flora can be prevented or improved.

### Brief Description of the Drawings

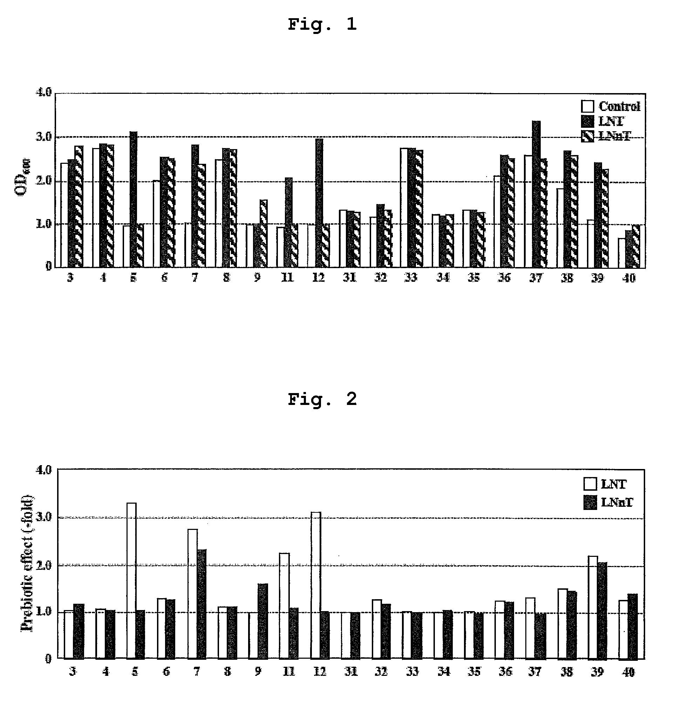

Fig. 1 shows the measured values of turbidity at wavelength 600 nm (OD₆₀₀) when various microorganisms belonging to the genus Bificobacterium were cultured for a given time in the presence of LNT or LNnT. In the Figure, an open (white) bar shows an oligosaccharide non-addition plot (control plot), a closed (black) bar shows a 0.5(w/v)% LNT addition test plot, and a bar with slant lines shows a 0.5(w/v)% LNnT addition test plot.
Fig. 2 shows prebiotic effects of LNT and LNnT for each microorganism and shows a value, OD₆₀₀ of oligosaccharide addition test plot/OD₆₀₀ of control plot, in Fig. 1. In the Figure, an open (white) bar shows the results of a 0.5(w/v)% LNT addition test plot, and a closed (black) bar shows the results of a 0.5(w/v)% LNnT addition test plot.

### Description of Embodiments

### 1. The composition of the present invention for promoting growth of a microorganism belonging to the genus Bifidobacterium

The composition of the present invention for promoting growth of a microorganism belonging to the genus Bifidobacterium contains one or more kinds selected from the group consisting of Lacto-N-tetraose (LNT), Lacto-N-neotetraose (LNnT) and derivatives thereof.

Lacto-N-tetraose (LNT; 4-O-[3-[2-(acetylamino)-2-deoxy-3-O-(β-D-galactopyranosyl)-β-D-glucopyranosyl]-β-D-galactopyranosyl]-D-glucose, Galβ1, 3GlcNAcβ1, 3Galβ1, 4Glc), and Lacto-N-neotetraose (LNnT; 4-O-[3-O-(4-O-β-D-galactopyranosyl-2-acetylamino-2-deoxy-β-D-glucopyranosyl)-β-D-galactopyranosyl]-α-D-glucopyranose, Galβ1, 4GlcNAcβ1, 3Galβ1, 4Glc) is neutral tetrasaccharide contained in breast milk of human and the like. LNT, LNnT and derivatives thereof used in the present invention may be chemically synthesized according to the method described in Carbohydrates in Chemistry and Biology (B. Ernst et al. ed., Willey-VCH, 2000) and the like, or produced according to the method described in WO 98/12343 and the like. It is also possible to use commercially available LNT and LNnT.

Examples of the derivatives of LNT and LNnT include a derivative wherein one or more hydroxyl groups thereof are alkylated by an alkyl group optionally having substituent(s) such as methyl, ethyl, hydroxypropyl, carboxymethyl and the like; a sulfated derivative wherein one or more hydroxyl groups are sulfated; a phosphorylated derivative wherein one or more hydroxyl groups are phosphorylated; a fucosylated derivative wherein fucose is bonded to one or more hydroxyl groups; a sialylated derivative wherein sialic acid is bonded to one or more hydroxyl groups; an aminated derivative wherein one or more hydroxyl groups are substituted by an amino group optionally substituted by an alkyl group, a pyridyl group and the like; a carboxylated derivative wherein one or more hydroxyl groups are substituted by a carboxyl group; a derivative wherein an acetylamino group is deacetylated; and the like. These can be produced by a method known per se.

Different from conventionally used bifidobacteria growth factors such as fructo-oligosaccharide, oligosaccharide and the like, LNT, LNnT and derivatives thereof are not digested by the host or other enteric bacteria. Therefore, they certainly arrive at the large intestine from the lower section of small intestine where microorganisms belonging to the genus Bifidobacterium live, and can efficiently promote the growth of the aforementioned microorganisms.

In addition, since LNT, LNnT and derivatives thereof are substances present in the living body, or derived from substances present in the living body, they have high affinity for living organisms and cause less safety problems.

The composition of the present invention for promoting growth of a microorganism belonging to the genus Bifidobacterium may directly contain one or more kinds selected from the group consisting of LNT, LNnT and derivatives thereof. Where necessary, it can also be provided by being contained in one or more kinds of pharmacologically acceptable carriers.

As the above-mentioned carrier, various carriers including water, alcohols such as ethanol and the like, fats and oils, gel, micell, emulsion, powder, capsule, liposome and the like can be used without limitation. The amount of one or more kinds selected from the group consisting of LNT, LNnT and derivatives thereof to be added to the carrier varies depending on the kind of the carrier to be used, the condition of the target who ingests and the like, and is not particularly limited as long as a sufficient growth-promoting effect is afforded on a microorganism belonging to the genus Bifidobacterium. It is generally about 0.001 wt% - 100 wt%, preferably 0.01 wt% - 100 wt%, more preferably 0.1 wt% - 100 wt%.

Where necessary, moreover, the composition of the present invention for promoting growth of a microorganism belonging to the genus Bifidobacterium may contain other component having a growth-promoting effect on the aforementioned microorganisms. Examples of such other component include disaccharides such as raffinose and the like, trisaccharides such as stachyose and the like, oligosaccharide mixtures such as lactooligosaccharide and the like, and the like.

The composition of the present invention for promoting growth of a microorganism belonging to the genus Bifidobacterium is produced by mixing one or more kinds selected from the group consisting of LNT, LNnT and derivatives thereof with the above-mentioned carriers as necessary, and by a method well known in the technical fields of pharmaceutical product, food, drink and the like, such as solubilization, dispersion, emulsification, kneading and the like.

Also, the composition of the present invention for promoting growth of a microorganism belonging to the genus Bifidobacterium can also be produced by molding and granulating by a method generally used in the technical fields of pharmaceutical product, food, drink and the like.

Examples of such molding and granulation methods include granulation methods such as fluidized bed granulation, stirring granulation, extrusion granulation, tumbling granulation, stream granulation, compression molding granulation, crush granulation, spray granulation, jet granulation and the like; coating methods such as pan coating, fluidized bed coating, dry coating and the like; swelling methods such as puff drying process, excessive steam method, foam mat method, microwave heating method and the like; extrusion methods using an extrusion granulator, an extruder and the like, and the like.

### 2. Growth promoter of microorganisms belonging to the genus Bifidobacterium

The composition of the present invention for promoting growth of a microorganism belonging to the genus Bifidobacterium can be provided as a growth promoter (drug for promoting growth) for the aforementioned microorganisms, which is specifically in the dosage form of tablet, powder, granule, pill, suspension, emulsion, infusion, capsule, liquid, syrup, elixir, extract, liquid extract, jelly and the like. To promote the growth of microorganisms belonging to the genus Bifidobacterium in the intestine, the aforementioned growth promoter is preferably administered orally and preferably provided in a dosage form suitable for oral administration.

Examples of the dosage form suitable for oral administration include liquid preparation such as liquid, syrup and suspension. These can contain a carrier or an additive for pharmaceutical products, for example, water; saccharides such as sucrose, sorbitol, fructose and the like; glycols such as polyethylene glycol, propylene glycol and the like; fats and oils such as sesame oil, olive oil, soybean oil and the like; preservatives such as paraoxybenzoates (e.g., methyl parahydroxybenzoate and the like), and the like; preservatives such as sodium benzoate and the like; flavors such as strawberry flavor, peppermint flavor and the like; and the like.

Examples of other dosage form suitable for oral administration include solid preparations such as tablet, powder, granule and the like. They can contain saccharides such as lactose, white soft sugar, glucose, sucrose and the like; sugar alcohols such as mannitol, sorbitol and the like; starches such as potato starch, wheat starch, corn starch and the like; inorganic salts such as calcium carbonate, calcium sulfate, sodium hydrogen carbonate, sodium chloride and the like; plant powders such as Glycyrrhiza uralensis powder, Gentiana lutea powder and the like; excipients such as crystalline cellulose, dextrin and the like; disintegrants such as agar, gelatin powder, carmellose sodium, carmellose calcium, sodium alginate and the like; lubricants such as magnesium stearate, talc, hydrogenated vegetable oil, macrogol, silicone oil and the like; binders such as polyvinyl alcohol, hydroxypropylcellulose, methylcellulose, ethylcellulose, carmellose, gelatin, starch paste and the like; surfactants such as sorbitan ester of fatty acid and the like; plasticizers such as glycerol and the like; and the like.

The above-mentioned growth promoter for microorganism belonging to the genus Bifidobacterium can be produced by mixing one or more kinds selected from the group consisting of LNT, LNnT and derivatives thereof with the above-mentioned carrier and additive for pharmaceutical products where necessary, and according to a method well known in the technical field of galenical pharmacy, for example, the method described in the Japanese Pharmacopoeia Preparation General Rules or pharmaceutical product production guidance and the like. That is, a liquid can be produced by adding one or more kinds selected from the group consisting of LNT, LNnT and derivatives thereof to a solvent such as water, ethanol and the like, which is followed by mixing and dissolving. A suspension is produced by adding the aforementioned active ingredient together with a suspending agent and other additives to water and suspending the mixture. An emulsion is produced by mixing with an oily component, water and an emulsifier and emulsifying and the mixture. A powder is produced by crystallizing and powderizing the aforementioned active ingredient, or mixing same with an additive such as excipient, binder, disintegrant and the like and powderizing or finely granulating the mixture. A granule is produced by crystallizing and powderizing the aforementioned active ingredient, or mixing same with an additive such as excipient, binder, disintegrant and the like to give a homogenized mixture, which is granulated and sieved. A hard capsule is produced by crystallizing and powderizing the aforementioned active ingredient, or mixing same with an additive such as excipient, binder, disintegrant and the like and granulating or molding the mixture, and filling same in a hard capsule. A soft capsule is produced by crystallizing or powderizing the aforementioned active ingredient, or mixing same with an additive such as excipient, binder, disintegrant and the like to give a homogenized mixture, each of which is cover-molded with a mixture of a capsule base such as gelatin and the like, glycerol or sorbitol and the like to increase plasticity. A tablet is produced by compression molding the granular growth promoter of the present invention, or moistening same with a solvent and casting same in a mold, which is followed by molding.

The above-mentioned granules and capsules can be processed into a sustained-release or enteric preparation by treating with a sustained-release or enteric film or with a sustained-release or enteric coating. Also, a tablet can be processed into a sugar-coated tablet, a film-coated tablet, a sustained-release tablet or an enteric-coated tablet.

A growth promoter of the above-mentioned microorganism belonging to the genus Bifidobacterium can be used for mammals such as human, domestic animals such as bovine, horse, sheep, swine and the like, pet animals such as dog, cat, monkey, guinea pig and the like, and the like, and particularly preferably used for the improvement of enteric bacterial flora in human adult. While the dose and administration frequency vary depending on the administration form, the species of the administration subject animal, age, sex, body weight, state of enteric bacterial flora and the like, the total amount of one or more kinds selected from the group consisting of LNT, LNnT and derivatives thereof is generally 1 mg - 50 g, preferably 5 mg - 20 g, per dose for oral administration, which is administered once to several times to a human adult (body weight 60 kg). When the aforementioned dose per administration is less than 1 mg, the aforementioned microorganism growth-promoting effect may not be achieved sufficiently. On the other hand, when the aforementioned dose per administration exceeds 50 g, the aforementioned microorganism growth-promoting effect may not be enhanced much, and formulation sometimes becomes difficult.

### 3. Food or drink for promoting growth of microorganisms belonging to the genus Bifidobacterium

The composition of the present invention for promoting growth of a microorganism belonging to the genus Bifidobacterium can also be provided as a food or drink for promoting growth of the aforementioned microorganisms. The aforementioned food or drink preferably contains one or more kinds selected from the group consisting of LNT, LNnT and derivatives thereof in an amount higher than that generally contained in foods and drinks.

Examples of the above-mentioned food and drink include drinks such as juice, beverages, soup, tea, lactobacillus beverages, fermentation milk and the like; milk products such as butter, cheese, yoghurt, processing milk, defatted milk powder and the like; meat processed products such as ham, sausage, hamburger and the like; processed seafood paste products such as *chikuwa, kamaboko* and the like; egg processed products such as *dashi-maki, tamago dofu* and the like; confectioneries such as frozen dessert, cookies, jelly, snack food, chewing gum and the like; bread; noodles; pickles; smoked products; dried fish; *tsukudani*; seasoning and the like.

While the form of the above-mentioned food or drink is not particularly limited, the form of, for example, solid such as powder, granule, sheet, capsule, tablet and the like; liquid such as solution, suspension, milky liquid and the like; fluent such as jelly and the like can be employed. It can be filled in a bottle, can, retort pouch or the like and provided in a tightly-sealed state, or contained in a box or bag or packaged with a box or bag and provided.

The above-mentioned food or drink is preferably used as a food for specified health uses, a food with health claims such as a food with nutrient function claims, or other dietary supplement, to promote growth of microorganisms belonging to the genus Bifidobacterium living in the intestine.

While the amount of the above-mentioned food or drink to be ingested is not particularly limited, the total amount of one or more kinds selected from the group consisting of LNT, LNnT and derivatives thereof is generally 0.1 g - 50 g, preferably 0.5 g - 20 g, per day, which is administered once to several times per day to a human adult (body weight 60 kg). While the food or drink of the present invention may be ingested only one day, to promote growth microorganism belonging to the genus Bifidobacterium in the intestine and maintain the enteric bacterial flora in a good state, continuous ingestion is preferable, and continuous ingestion is performed for 2 days - 5 years, preferably 2 weeks - 1 year. This ingestion amount is merely a guidance, and it can be adjusted to fall within an appropriate preferable range according to the state of enteric bacterial flora, age, sex, body weight and the like of the subject of ingestion.

According to the food or drink for promoting growth of a microorganism belonging to the genus Bifidobacterium, a food or drink containing the above-mentioned single ingestion amount of one or more kinds selected from the group consisting of LNT, LNnT and derivatives thereof can be provided as a unit package serving form. The unit package serving form" refers to a form for which the amount to be taken per serving is defined in advance. For example, in the case of drink, chewing gum, jelly, yoghurt, cookie and the like, a form wherein a given amount is packed in a container such as a pack, a bag, a bottle, a box and the like can be mentioned; and in the case of foods such as granule, powder, slurry and the like, a form wherein an amount to be taken per serving is packed in a pack, a bag, and the like can be mentioned. Particularly, when the food or drink is a food for specified health uses, a food with health claims such as a food with nutrient function claims and the like, other nutrition aid food and the like, for example, a form wherein a food or drink containing a single ingestion amount of LNT and the like is packed in a unit amount to be ingested per meal, a form wherein a drink wherein a single ingestion amount of LNT and the like is suspended or dissolved is filled in a bottle and the like for a single consumption can be mentioned.

The above-mentioned food or drink may contain various carriers and, where necessary, additives generally used for foods and drinks, for example, additives such as sweetener, bittering agent, acidulant, seasoning, colorant, color former, bleaching agent, preservative, fungicide, antioxidant, thickening agent, gum base, enzyme, brightening agent, emulsifier, reinforcement, production material, flavor, spice and the like described in food additive handbook (Japan Food Additive Association, published on January 6, 1997) may be added.

Examples of such additive include the following.

Examples of the sweetener include aspartam, licorice, stevia, xylose, luo han guo (Siraitia grosvenorii) and the like. Examples of the bittering agent include iso-alpha bitter acid, olive tea, caffeine, turkey tail extract, Cinchona extract, amur cork extract, Gentiana lutea extract, spice extract, enzyme treatment naringin, Jamaica quassia extract, bitter orange extract, theobromine, naringin, bitterwood extract, Quassia extract, Rabalosia japonica extract, Agaricus blazei extract, hop extract, Brotowali, methylthioadenosine, artemisia extract, lycee extract and the like. Examples of the acidulant include adipic acid, itaconic acid, citric acid and a salt thereof, succinic acid and a salt thereof, sodium acetate, tartaric acid and a salt thereof, carbon dioxide, lactic acid, phytic acid, fumaric acid, malic acid, phosphoric acid and the like. Examples of the seasoning include amino acids such as asparagine, aspartic acid, glutamic acid, glutamine, alanine, isoleucine, glycine, serine, cystine, tyrosine, leucine, proline and the like; nucleic acids such as disodium 5'-inosinate, disodium 5'-uridylate, disodium 5'-guanylate, disodium 5'-cytidylate, calcium 5'-ribonucleotide, disodium 5'-ribonucleotide and the like; inorganic salts such as potassium chloride, sodium chloride-decreased brine, crude potassium chloride (sea water), Whey salt, tripotassium phosphate, dipotassium hydrogen phosphate, potassium dihydrogen phosphate, disodium hydrogen phosphate, sodium dihydrogen phosphate, trisodium phosphate and the like; chlorella extract and the like.

Examples of the colorant include carotenoid, turmeric dye, flavonoid, caramel dye, Lithospermum root dye, Spirulina dye, chlorophyl, murasakiimo dye, murasakiyamaimo dye, Japanese basil dye, blueberry dye and the like. Examples of the color former include sodium nitrite and the like. Examples of the bleaching agent include sodium sulfite and the like.

Examples of the preservative include sodium sulfite, benzoic acid and a salt thereof, udo extract, storax extract, A. capillaries Thunb extract, sorbic acid and a salt thereof, propionic acid and a salt thereof and the like. Examples of the fungicide include orthophenylphenol and the like.

Examples of the antioxidant include ascorbic acid, a salt and a derivative thereof, ethylenediamine tetrasodium acetate, calcium ethylenediaminetetraacetate, erythorbic acid, oryzanol, catechin, clove extract, quercetin, enzyme treatment rutin, enzyme decomposition apple extract, pepper extract, sesame oil unsaponifiable matter, dibutylhydroxytoluene, essential oil-removed fennel extract, horseradish extract, Japanese parsley extract, tea extract, tempeh extract, Houttuynia cordata extract, tocotrienol, tocopherol and an ester thereof, rape seed oil extract, green coffee bean extract, sunflower seed extract, ferulic acid, butylhydroxyanisole, blueberry leaf extract, propolis extract, tree fern·gingko extract, hesperetin, Impatiens balsamina extract, gallic acid, myrica extract, eucalyptus extract, rosemary extract and the like.

Examples of the thickening agent include polysaccharides and derivatives thereof such as gum arabic, xanthan gum, alginic acid and a salt thereof, chitin, chitosan, guargum, hydroxypropylcellulose, corn starch, carboxymethylcellulose and a salt thereof, agar, dextrin, methylcellulose, microfibrouscellulose, crystalline cellulose, seaweed cellulose, carageenan, mannan and the like; proteins such as casein sodium, gelatin and the like; polymer compounds such as polyvinyl alcohol, sodium polyacrylate, sodium polyphosphate and the like; natural polymers such as Aloe arborescens extract, yeast cell wall, konnyakuimo extract, nata de coco and the like; and the like.

Examples of the gum base include methyl acetylricinoleate, Urushi wax, gum ester, elemiresin, Urucury wax, ozocerite, opopanax resin, kauri gum, Carnauba wax, guaiacresin, Gutta katiau, Palaquium leiocarpum, Gutta Percha, spermaceti wax, Copaiba Balsam, copal resin, rubber, Rice bran wax, sugar cane wax, shellac, jelutong, solver, talc, calcium carbonate, dammar resin, chicle, chilte, Castilla fallax, low molecule rubber, paraffin wax, Balsam Fir, propylene glycol ester of fatty acid, pulp powder, chaff powder, jojoba wax, polyisobutylene, polybutene, microcrystalline wax, mastic, Manilkara solimoesensis, beeswax and the like.

Examples of the enzyme include amylase, trypsin, rennet and the like.

Examples of the brightening agent include Urushi wax, rhus succedanea fruit wax and the like.

Examples of the emulsifier include glycerol fatty acid ester, enzyme treated lecithin, sucrose fatty acid ester, sorbitan fatty acid ester, soybean saponin, polyglycerol fatty acid ester, polysorbates, egg-yolk lecithin and the like.

Examples of the reinforcement include zinc salt, various amino acid, 5-adenyl acid, iron chloride, enzyme treatment hesperidin, various calcined calciums, various unbaked calciums, dibenzoylthiamine, calcium hydroxide, calcium carbonate, thiaminehydrochloride, dunaliella carotene, nicotinic acid, carrot carotene, palm oil carotene, calcium pantothenate, vitamin A, hydroxyproline, pyrrocalcium dihydrogen phosphate, ferrous pyrophosphate, ferric pyrophosphate, ferritin, heme iron, menaquinone, folic acid, riboflavin and the like.

Examples of the production material include processing aids such as acetone, ion exchange resin and the like, ficus extract, rice straw ash extract, ethanol, kaolin, glycerol fatty acid ester, mulberry extract, bone ash, Japanese basil extract, ginger extract, various tannin, pfaffia extract, grapes seed extract, calcium phosphate and the like.

Examples of the flavor and spice include cinnamomi cortex acid, citral, citronellal, vanilla flavoring, d-borneol, 1-menthol and the like.

### 4. Food additive for promoting the growth of microorganisms belonging to the genus Bifidobacterium

Furthermore, the composition of the present invention for promoting growth of a microorganism belonging to the genus Bifidobacterium can be provided as a food additive for promoting the growth of the aforementioned microorganisms. The aforementioned food additive can be produced by a conventional production method of food additives. The aforementioned food additive can also contain the carriers described in the above-mentioned 1., and general additives for food or drink described in the above-mentioned 3.

The above-mentioned food additive preferably contains one or more kinds selected from the group consisting of LNT, LNnT and derivatives thereof in such amount that provides the final content of the aforementioned LNT and the like in a food or drink, when the food additive is added to the food or drink, that permits intake of the above-mentioned preferable ingestion amount of LNT and the like in a food or drink for promoting the growth of a microorganism belonging to the genus Bifidobacterium. Therefore, the content of LNT and the like in the above-mentioned food additive is preferably 0.001 wt% - 100 wt% as the total amount of LNT and the like.

The composition of the present invention for promoting growth of a microorganism belonging to the genus Bifidobacterium can also be provided as a feed or feed additive for domestic animals and pet animals.

Ingestion of the composition of the present invention for promoting growth of a microorganism belonging to the genus Bifidobacterium enables promotion of the growth of the aforementioned microorganisms in the intestine of a mammal, particularly human.

Particularly, the composition of the present invention for promoting growth of a microorganism belonging to the genus Bifidobacterium is suitable for human adult. Preparation of the composition for promoting growth of the aforementioned microorganisms for adult and ingestion thereof by adults can promote growth of one or more kinds selected from the group consisting of bifidobacteria, particularly microorganisms belonging to B. bifidum and microorganisms belonging to B. longum, which are living in the intestine of adult and decreasing with aging.

Accordingly, the subject to be ingested with the composition of the present invention for promoting growth of the above-mentioned microorganisms is a human showing a tendency toward a decrease with aging in the microorganisms belonging to the genus Bifidobacterium indigenous to the intestine, who is preferably 20 years old or older, more preferably 25 years old or older, further preferably 30 years old or older, more preferably 50 years old or older. Particularly preferable subject is a human who is preferably 20 years old or older and showing a decreased living ratio of microorganisms belonging to the genus Bifidobacterium, particularly a microorganism belonging to B. bifidum or a microorganism belonging to B. longum, relative to the enteric bacterium group, as compared to the average. A decreased living ratio of the aforementioned microorganism relative to the enteric bacterium group can be produced by constipation and diarrhea caused by various factors, as well as microbial substitution and the like possibly due to the use of an antibacterial substance.

Therefore, ingestion of the composition of the present invention for promoting growth of a microorganism belonging to the genus Bifidobacterium can prevent or improve various symptoms caused by abnormality in the enteric bacterial flora. Examples of the various symptoms caused by abnormality in the enteric bacterial flora include bowel movement disorders such as constipation and diarrhea, abdominal distension due to intestinal abnormal fermentation, antibacterial substance-induced colitis and the like.

Furthermore, the present invention can provide a commercial package comprising the composition of the present invention for promoting growth of a microorganism belonging to the genus Bifidobacterium, and a written matter stating that the composition can or should be used for promoting growth of a microorganism belonging to the genus Bifidobacterium.

As the commercial package of the present invention, a pharmaceutical product package comprising the growth promoter of a microorganism belonging to the genus Bifidobacterium of the present invention, and the aforementioned written matter, a food or drink package comprising the food or drink of the present invention for promoting growth of a microorganism belonging to the genus Bifidobacterium and the aforementioned written matter, and the like can be mentioned.

In the aforementioned written matter, it is described that the composition of the present invention for promoting growth of a microorganism belonging to the genus Bifidobacterium promotes growth of a microorganism belonging to the genus Bifidobacterium indigenous to the intestine of, particularly, an adult, and is effective for the prophylaxis or improvement of various symptoms caused by abnormality in the enteric bacterial flora, and provides explanation on applicable specific conditions of symptoms developed by abnormality in the enteric bacterial flora, administration or ingestion subject, single dose or ingestion amount, administration or ingestion frequency per day, administration or ingestion method and the like.

### Examples

The present invention is explained in detail in the following by referring to Examples, which are not to be construed as limitative.

### [Example 1] Production of growth promoter containing LNT or LNnT, for microorganism belonging to the genus Bifidobacterium (1)

Based on the following composition, each component was mixed and homogenized to give the above-mentioned growth promoter.

### <composition>

| | |
|---|---|
| LNT or LNnT (produced by the method described in WO 98/12343) | 49 g |
| Pinedex#3 (excipient; manufactured by Matsutani Chemical Co., Ltd.) | 49 g |
| Ferric pyrophosphate (iron source; manufactured by Yoneyama chemical Industry Co., Ltd.) | 0.1 g |
| HOSUKARU EFC (calcium source; manufactured by Nikko Fine Products Co., Ltd.) | 1 g |
| vitamin mix (manufactured by Merck & Co., Inc.) | 1 g |

### [Example 2] Production of growth promoter containing LNT or LNnT, for microorganism belonging to the genus Bifidobacterium (2)

LNT or LNnT (produced by the method described in WO 98/12343) (20 g) was dispersed in water (180 mL) to give the above-mentioned growth promoter.

### [Example 3] Production of drink containing LNT or LNnT, for promoting growth of microorganism belonging to the genus Bifidobacterium

LNT (produced by the method described in WO 98/12343) (0.64 kg), LNnT (produced by the method described in WO 98/12343) (0.64 kg), erythritol (manufactured by Mitsubishikagaku Foods Corporation) (3 kg), citric acid (manufactured by Iwata Chemical Co., Ltd.) (0.05 kg), an artificial sweetener (sucralose; manufactured by San-Ei Gen F.F.I., Inc.) (3 g), and a flavor (lemone essence; manufactured by Nippon Flavour Kogyo Co., Ltd.) (0.06 kg) were dissolved by stirring in water (50 L) at a liquid temperature of 70°C. The solution was adjusted to pH 3.3 with citric acid (manufactured by Iwata Chemical Co., Ltd.), and sterilized by plate sterilization. Then, the solution is filled in a bottle and pasteurizer sterilized to give the above-mentioned drink.

### [Experimental Example] Growth-promoting effect of LNT and LNnT on microorganism belonging to the genus Bifidobacterium 1. Obtainment of various microorganisms belonging to the genus Bifidobacterium

Table 1 shows the microorganisms belonging to the genus Bifidobacterium and used in the Experimental Example. The microorganisms were purchased from independent administrative agency RIKEN Tsukuba Laboratory BioResource Center (3-1-1 Koyadai, Tsukuba, Ibaraki 305-0074, Japan). As Bifidobacterium longum subsp. longum NCC2705, the strain preserved under GenBank accession number NC004307 was used.

**Table 1**

| No. | name of strain | RIKEN BioResource Center No. |
|---|---|---|
| 3 | *Bifidobacterium breve* | 1192 |
| 4 | *Bifidobacterium pseudolongum* subsp. *Pseudolongum* | 1205 |
| 5 | *Bifidobacterium longum* subsp. *Longum* | 1217 |
| 6 | *Bifidobacterium longum* subsp. *Infantis* | 1222 |
| 7 | *Bifidobacterium bifidum* | 1254 |
| 8 | *Bifidobacterium bifidum* | 7004 |
| 9 | *Bifidobacterium bifidum* | 1255 |
| 11 | *Bifidobacterium longum* subsp. *Longum* | NCC2705 |
| 12 | *Bifidobacterium longum* subsp. *Longum* | 7054 |
| 31 | *Bifidobacterium adolescentis* | 1275 |
| 32 | *Bifidobacterium animalis* subsp. *Lactis* | 10602 |
| 33 | *Bifidobacterium gallicum* | 8224 |
| 34 | *Bifidobacterium catenulatum* | 1194 |
| 35 | *Bifidobacterium scardovii* | 12489 |
| 36 | *Bifidobacterium angulatum* | 7096 |
| 37 | *Bifidobacterium pseudocatenulatum* | 1200 |
| 38 | *Bifidobacterium dentium* | 1195 |
| 39 | *Bifidobacterium longum* subsp. *Infantis* | 1210 |
| 40 | *Bifidobacterium adolescentis* | 7046 |

### 2. Evaluation of growth-promoting effect of LNT and LNnT on microorganism belonging to the genus Bifidobacterium

Glycerol stock of each strain shown in Table 1 was plated in GAM bouillon (manufactured by Nissui Pharmaceutical Co., Ltd.) agar medium, and cultured using a gas pack ("Anaeropack", manufactured by Mitsubishi Gas Chemical Company, Inc.), under anaerobic conditions at 37°C for 2 days to renature the test strain. Each strain was inoculated in 5 mL GAM bouillon liquid medium, and precultured in the same manner as above under an anaerobic condition at 37°C for 18 hr.

The turbidity (OD₆₀₀) of each culture medium was measured at wavelength 600 nm on completion of the preculture. The preculture medium was seeded in 1 mL of main test liquid medium such that the OD₆₀₀ value at the time of start of the culture was 0.010, the cells were cultured under anaerobic conditions at 37°C for 24 hr in the same manner as above, and OD₆₀₀ of the culture medium was measured on completion of the culture. The liquid medium for the test was prepared by adding LNT or LNnT to the GAM bouillon liquid medium at a final concentration of 0.5(w/v)%. In addition, GAM bouillon liquid medium free of both oligosaccharides LNT, LNnT was used as a control. The measurement results of OD₆₀₀ of the culture medium on completion of the culture are shown in Fig. 1. To evaluate the growth-promoting effect (prebiotic effect) of LNT and LNnT, the relative value of the oligosaccharide (LNT or LNnT) addition plot, when the measurement value OD₆₀₀ of the oligosaccharide non-addition plot (control) was 1, is shown in Fig. 2.

As is clear from Figs. 1 and 2, LNT showed a strong growth-promoting effect (prebiotic effect = 2-fold or more) on Bifidobacterium longum subsp. Longum 1217 strain, Bifidobacterium bifidum 1254 strain, Bifidobacterium longum subsp. Longum NCC2705 strain, Bifidobacterium longum subsp. Longum 7054 strain, and Bifidobacterium longum subsp. Infantis 1210 strain, and LNnT showed a strong growth-promoting effect (prebiotic effect = 2-fold or more) on Bifidobacterium bifidum 1254 strain, and Bifidobacterium longum subsp. Infantis 1210 strain.

### Industrial Applicability

As described in detail above, according to the present invention, the growth of microorganisms belonging to the genus Bifidobacterium, which are good bacteria living in the intestine, can be promoted effectively by a convenient means of oral ingestion. As a result, enteric bacterial flora can be improved to a good state, and the state can be maintained, and bowel diseases and various symptoms caused by changes in the enteric bacterial flora can be prevented or improved.

This application is based on a patent application No. 2012-098224 filed in Japan (filing date: April 23, 2012), the contents of which are incorporated in full herein.

## Claims

1. A composition for promoting growth of a microorganism belonging to the genus Bifidobacterium, comprising one or more kinds selected from the group consisting of Lacto-N-tetraose, Lacto-N-neotetraose and derivatives thereof.

2. The composition according to claim 1, wherein the microorganism belonging to the genus Bifidobacterium is one or more kinds selected from the group consisting of a microorganism belonging to Bifidobacterium bifidum and a microorganism belonging to Bifidobacterium longum.

3. The composition according to claim 1 or 2, which promotes the growth of a microorganism belonging to the genus Bifidobacterium in the intestine of human adult.

4. The composition according to any one of claims 1 to 3, which is a growth promoter of a microorganism belonging to the genus Bifidobacterium.

5. The composition according to any one of claims 1 to 3, which is a food or drink.

6. The composition according to claim 5, wherein the food or drink is a food with health claims or dietary supplement.

7. The composition according to any one of claims 1 to 3, which is a food additive.

8. Use of one or more kinds selected from the group consisting of Lacto-N-tetraose, Lacto-N-neotetraose and derivatives thereof for the production of a composition for promoting growth of a microorganism belonging to the genus Bifidobacterium.

9. A method of promoting growth of a microorganism belonging to the genus Bifidobacterium in a subject in need thereof, comprising orally administering a composition containing one or more kinds selected from the group consisting of Lacto-N-tetraose, Lacto-N-neotetraose and derivatives thereof to the subject.

10. The method according to claim 9, wherein the microorganism belonging to the genus Bifidobacterium is one or more kinds selected from the group consisting of a microorganism belonging to Bifidobacterium bifidum and a microorganism belonging to Bifidobacterium longum.

11. The method according to claim 9 or 10, which promotes growth of a microorganism belonging to the genus Bifidobacterium in the intestine of human adult.

12. A composition comprising one or more kinds selected from the group consisting of Lacto-N-tetraose, Lacto-N-neotetraose and derivatives thereof for use in promoting growth of a microorganism belonging to the genus Bifidobacterium.

13. A commercial package comprising a composition for promoting growth of a microorganism belonging to the genus Bifidobacterium, which contains one or more kinds selected from the group consisting of Lacto-N-tetraose, Lacto-N-neotetraose and derivatives thereof, and a written matter with an explanation relating to use for promoting growth of a microorganism belonging to the genus Bifidobacterium.
